# EUROPÄISCHE PATENTSCHRIFT

(11) **EP 2 069 283 B1**
(45) Veröffentlichungstag und Bekanntmachung des Hinweises auf die Patenterteilung: **30.06.2010**
(21) Anmeldenummer: 07820210.8
(22) Anmeldetag: 14.09.2007
(51) Int. Cl.: C07C 209/36, B01J 8/18

(54) **VERFAHREN ZUR HERSTELLUNG VON AROMATISCHEN AMINEN IN EINEM WIRBELSCHICHTREAKTOR**
METHOD FOR THE PRODUCTION OF AROMATIC AMINES IN A FLUIDIZED BED REACTOR
PROCÉDÉ POUR PRODUIRE DES AMINES AROMATIQUES DANS UN RÉACTEUR À LIT FLUIDISÉ

(30) Priorität: 19.09.2006 EP 06120885
(43) Veröffentlichungstag der Anmeldung: 17.06.2009
(73) Patentinhaber: BASF SE, 67056 Ludwigshafen (DE)
(72) Erfinder: SEIDEMANN, Lothar, 68169 Mannheim (DE); KÖNIGSMANN, Lucia, 76227 Karlsruhe (DE); SCHNEIDER, Christian, 68165 Mannheim (DE); SCHWAB, Ekkehard, 67434 Neustadt (DE); STÜTZER, Dieter, 67373 Dudenhofen (DE); LIEKENS, Celine, B-2650 Edegem (BE)
(86) Internationale Anmeldenummer: PCT/EP2007/059703
(87) Internationale Veröffentlichungsnummer: WO 2008/034770

(56) Entgegenhaltungen:
- EP-A- 0 331 465
- EP-A- 0 428 265
- EP-A- 1 477 224
- WO-A-2005/077520
- DE-A1- 10 226 120
- DE-A1-102004 014 677
- DE-B- 1 114 820
- US-A- 3 429 654
- US-A- 3 482 946
- DIAO, S. ET AL.: "Gaseous catalytic hydrogenation of nitrobenzene to aniline in a two-stage fluidized bed reactor" APPLIED CATALYSIS A: GENERAL, Bd. 286, Nr. 1, 2005, Seiten 30-35, XP004897383 ISSN: 0926-860X

## Beschreibung

Die Erfindung betrifft ein Verfahren zur Herstellung von aromatischen Aminen durch katalytische Hydrierung der entsprechenden Nitroverbindungen in einem Wirbelschichtreaktor, insbesondere die Herstellung von Anilin durch katalytische Hydrierung von Nitrobenzol.

Aus DE-A 2 849 002 ist ein Verfahren zur katalytischen Gasphasen-Hydrierung von aromatischen Aminen an Palladium-haltigen Trägerkatalysatoren bekannt, das bevorzugt in Rohrreaktoren durchgeführt wird, wobei die Reaktionswärme mit einer geeigneten Wärmeträgerflüssigkeit, deren Temperatur in einem Bereich von etwa 150 bis 350 °C gehalten wird, abgeführt wird. Nachteilig ist die begrenzte mögliche Wärmeabfuhr: Die möglichen Wärmestromdichten für die Abfuhr von Reaktionswärme durch einen Wärmeübertrager liegen für ortsfest angebrachte Trägerkatalysatoren im Bereich von 0,5 bis 5 kW pro Quadratmeter, so dass bei stark exothermen Reaktionen, speziell die Hydrierung von Nitrobenzol zu Anilin, die Abführung der Reaktionswärme bei Verwendung eines Rohrreaktors auf Schwierigkeiten stößt.

Entsprechend wurde vorgeschlagen, die Hydrierung zu aromatischen Aminen in Wirbelschichtreaktoren durchzuführen, die für ihre sehr guten Wärmeübergangseigenschaften bekannt sind. Derartige Verfahren sind beispielsweise in DE-A 1 114 820 und DE-A 1 133 394 beschrieben: Als Hydrierkatalysatoren kommen nach den genannten Druckschriften die Schwermetalle der 1. und 5. bis 7. Gruppe des Periodensystems sowie der Eisen- und Platingruppe, beispielsweise Kupfer, Molybdän, Wolfram, Nickel, Kobalt oder Gemische hiervon, also wie ihre Oxide, Sulfide oder Halogenide, gegebenenfalls zusammen mit Bor oder Bor-Verbindungen, in Betracht. Die Katalysatoren können auf unterschiedliche Träger aufgebracht sein. Die Katalysatoren weisen eine geringe Korngröße auf, beispielhaft von 0,3 mm, und werden durch das zu hydrierende Ausgangsgemisch und den für die Hydrierung notwendigen Wasserstoff, gegebenenfalls in Mischung mit einem Inertgas, in wirbelnder Bewegung gehalten.

Im Verfahren des Hauptpatentes DE-A 1 114 820 wird drucklos gearbeitet, abweichend hiervon im Verfahren des Zusatzpatentes DE-A 1 133 394, bei erhöhtem Druck von mindestens 3 Atmosphären Überdruck, wobei eine längere Lebensdauer des Katalysators erreicht werden soll.

Aufgrund der sehr guten Wärmeabfuhreigenschaften des Wirbelbettes, wo zur Abfuhr von Reaktionswärme Wärmestromdichten im Bereich von 10 bis 100 kW pro Quadratmeter realisiert werden können, kann der Wirbelbettreaktor für die favorisierte isotherme Reaktionsführung deutlich einfacher im Vergleich zu Rohrreaktoren gestaltet werden, die aufwändig gekühlt werden müssen.

Die US-A-3 429 654 beschreibt ein Verfahren zur Hydrierung von Nitrobenzol zu Anilin in einem Wirbelschichtreaktor mit Hohlkugeln, die beispielhaft einen Durchmesser von 15 mm und 10 kreisförmige Öffnungen, die gleichförmig über die Oberfläche der Kugeln verteilt sind, mit Durchmessern der Öffnungen von 5 mm, aufweisen.

Der Fachartikel aus Applied Catalysis, Band 286, Nr. 1, 2005, Seiten 30 bis 35 (Diao, S. et al.: Gaseous catalytic hydrogenation of nitrobenzene to aniline i a two-stage fluidized bed reactor) beschreibt ein Verfahren zur katalytischen Hydrierung von Nitrobenzol zu Anilin in einem Wirbelschichtreaktor, worin ein Lochboden mit einem sehr kleinen Öffnungsverhältnis im axialen mittleren Bereich eingebaut ist, wodurch zwei Reaktionsräume gebildet werden, zwischen denen eine Rückvermischung verhindert wird. Dadurch wird der Umsatz an Nitrobenzol und insbesondere die Lebensdauer des Katalysators erhöht. Das Dokument beschreibt nicht den Einbau von Zellen in einem Wirbelschichtreaktor für die katalytische Hydrierung von Nitroverbindungen zu den entsprechenden Aminen.

Die US 3,482,946 beschreibt einen Wirbelschichtreaktor, der mittels Lochböden in mehrere Reaktionsräume abgeteilt ist.

Die WO 2005/077520 beschreibt einen Wirbelschichtreaktor, wobei in der Wirbelschicht gasdurchlässige Platten angeordnet sind, die mit einem in der Wirbelschicht angeordneten Wärmeübertrager wärmeleitend verbunden sind. Dabei soll die Größe der Öffnungen bzw. die Struktur der gasdurchlässigen Platten so ausgelegt sein, dass eine Koaleszenz der Gasblasen vermieden wird, mit der Folge einer besseren Durchmischung der Wirbelschicht und dadurch einer gleichmäßigeren Temperaturverteilung.

Die DE-A 10 2004 014 677, beschreibt einen Wirbelschichtreaktor zur Durchführung exothermer chemischer Gleichgewichtsreaktionen, wobei zur Steuerung der Temperaturverteilung mindestens ein Wärmetauscher im Wirbelbett angeordnet ist. Die Wirbelschicht ist nicht in Zellen unterteilt.

Die EP-A 0 331 465 beschreibt ein Verfahren zur Herstellung von Chlor in einen Wirbelschichtreaktor, worin eine Vielzahl von Lochböden angeordnet sind. Die Wirbelschicht ist nicht durch Zellen unterteilt.

Die EP-A 1 477 224, EP-A 0 428 265 und DE-A 102 26 120 beschreiben Kreuzkanalpackungen zum Einsatz in Verfahren für den Stoff- und/oder Wärmeaustausch.

Als nachteilig erweist sich die Wirbelschicht jedoch hinsichtlich des Stoffübergangs, da durch die Bildung von feststoffarmen Gasblasen der Kontakt zwischen Katalysator und Reaktionspartnern in bekannter Weise limitiert ist. Dies hat zur Folge, dass ein Teil der aromatischen Nitroverbindungen nicht in Kontakt mit dem wirbelnden Trägerkatalysator kommt und er die Reaktionszone nicht umgesetzt verlässt. Dadurch sinkt nicht nur der Umsatz, sondern es ergeben sich auch weitere Nachteile: Beispielsweise erweist sich nicht umgesetztes Nitrobenzol im Anilin störend bei der Herstellung von Diphenylmethandiisocyanat (MDI), das ein wichtiges Zwischenprodukt in der Polyurethan-Wertschöpfungskette ist.

Es war daher Aufgabe der Erfindung, das Verfahren zur Herstellung von aromatischen Aminen durch katalytische Hydrierung der entsprechenden Nitroverbindungen in einem Wirbelschichtreaktor weiter zu verbessern.

Die Aufgabe wird gelöst durch ein Verfahren zur Herstellung von aromatischen Aminen in einem Wirbelschichtreaktor durch katalytische Hydrierung der entsprechenden Nitroverbindung, wobei ein gasförmiges Reaktionsgemisch, enthaltend die Nitroverbindung und Wasserstoff, einen heterogenen partikelförmigen, eine Wirbelschicht ausbildenden Katalysator von unten nach oben durchströmt und wobei in der Wirbelschicht Einbauten vorgesehen sind, die die Wirbelschicht in eine Mehrzahl von horizontal sowie eine Mehrzahl von vertikal im Wirbelschichtreaktor angeordneten Zellen aufteilen, mit Zellwänden, die gasdurchlässig sind und die Öffnungen aufweisen, die eine Austauschzahl des heterogenen, partikelförmigen Katalysators in vertikaler Richtung im Bereich von 1 bis 100 Litern/Stunde pro Liter Reaktorvolumen gewährleisten, das dadurch gekennzeichnet ist, dass die Einbauten als Kreuzkanalpackung ausgebildet sind, mit in vertikaler Richtung im Wirbelbettreaktor parallel zueinander angeordneten geknickten gasdurchlässigen Metallblechen, Streckmetall- oder Gewebelagen, mit Knickkanten, die Knickflächen mit einem von Null verschiedenen Neigungswinkel zu Vertikalen ausbilden, und wobei die Knickflächen aufeinander folgender Metallbleche, Streckmetalll- oder Gewebelagen den gleichen Neigungswinkel, jedoch mit umgekehrten Vorzeichen aufweisen und dadurch Zellen ausbilden, die in vertikaler Richtung durch Engstellen zwischen den Knickkanten begrenzt werden.

Es wurde gefunden, dass es wesentlich ist, die Wirbelschicht mittels Einbauten sowohl in horizontaler als auch in vertikaler Richtung in Zellen aufzuteilen, das heißt in von Zellwänden umschlossene Hohlräume, wobei die Zellwände gasdurchlässig sind und Öffnungen aufweisen, die in vertikaler Richtung im Wirbelschichtreaktor einen Feststoffaustausch erlauben. Darüber hinaus können in den Zellwänden Öffnungen vorgesehen sein, die einen Feststoffaustausch in horizontaler Richtung erlauben. Der heterogene partikelförmige Katalysator kann sich somit zwar in vertikaler Richtung und gegebenenfalls auch in horizontaler Richtung durch den Wirbelschichtreaktor bewegen, er wird aber in den einzelnen Zellen im Vergleich zu einer Wirbelschicht ohne dieselben zurückgehalten, wobei die oben definierten Austauschzahlen gewährleistet werden.

Die Austauschzahl wird durch den Einsatz von radioaktiv markierten Feststoff-Tracer-Partikeln bestimmt, die in das wirbelnde Reaktionssystem eingegeben werden, wie beispielsweise in: G. Reed "Radioisotope techniques for problem-solving in industrial process plants", Chapter 9 ("Measurement of residence times and residence-time distribution"), p. 112-137, (J.S. Charlton, ed.), Leonard Hill, Glasgow and London 1986, (ISBN 0-249-44171-3), beschrieben. Über die Aufnahme von Zeit und Ort dieser radioaktiv markierten Partikel kann die Feststoffbewegung lokal bestimmt und die Austauschzahl abgeleitet werden (G. Reed in: "Radioisotope techniques for problem-solving in industrial process plants", Chapter 11 ("Miscellanous radiotracer applications", 11.1."Mixing and blending studies"), p. 167-176, (J. S. Charlton, ed.), Leonard Hill, Glasgow and London 1986, (ISBN 0-249-44171-3).

Durch die gezielte Auswahl der Geometrie der Zellen kann die Verweilzeit des heterogenen partikelförmigen Katalysators in denselben an die Charakteristik der jeweils durchzuführenden Reaktion angepasst werden.

Durch die Reihenschaltung einer Mehrzahl, das heißt insbesondere von 0 bis 100 Zellen oder auch von 10 bis 50 Zellen pro Meter Betthöhe, das heißt in vertikaler Richtung, in Richtung der Gasströmung von unten nach oben durch den Reaktor, wird die Rückvermischung begrenzt und damit die Selektivität und der Umsatz verbessert. Durch die zusätzliche Anordnung einer Mehrzahl von, das heißt von 10 bis 100 Zellen oder auch von 10 bis 50 Zellen pro Meter in horizontaler Richtung im Wirbelschichtreaktor, das heißt von Zellen, die parallel oder in Serie vom Reaktionsgemisch durchströmt werden, kann die Kapazität des Reaktors nach Bedarf angepasst werden. Die Kapazität des erfindungsgemäßen Reaktors ist somit nicht begrenzt und kann dem konkreten Bedarf angepasst werden, beispielsweise auch für Reaktionen im großtechnischen Maßstab.

Indem die Zellen Hohlräume umschließen, die den partikelförmigen heterogenen Katalysator aufnehmen, nimmt das Zellmaterial selbst nur einen begrenzten Teil des Querschnitts des Wirbelbettreaktors ein, insbesondere von nur etwa 1 bis 10 % der Querschnittsfläche des Wirbelschichtreaktors und führt daher nicht zu den bei den Einbauten aus dem Stand der Technik bekannten, mit einer erhöhten Querschnittsbelegung verbundenen Nachteilen.

Der im erfindungsgemäßen Verfahren eingesetzte Wirbelschichtreaktor wird, wie üblich, von unten über einen Gasverteiler mit den gasförmigen Einsatzstoffen beaufschlagt. Beim Passieren der Reaktionszone werden die gasförmigen Einsatzstoffe an dem heterogenen partikelförmigen Katalysator, der durch die Gasströmung zum Wirbeln angeregt wird, partiell umgesetzt. Die partiell umgesetzten Einsatzstoffe strömen in die nächste Zelle; wo dieselben weiter partiell umgesetzt werden.

Oberhalb der Reaktionszone ist eine Feststoffabscheidevorrichtung vorgesehen, die mitgeführten Katalysator von der Gasphase trennt. Das umgesetzte Produkt verlässt den erfindungsgemäßen Wirbelschichtreaktor am oberen Ende desselben feststofffrei.

Darüber hinaus kann der erfindungsgemäß eingesetzte Wirbelschichtreaktor sowohl von unten als auch von der Seite zusätzlich mit flüssigen Edukten beaufschlagt werden. Diese müssen jedoch direkt an ihrer Zugabestelle verdampfen können, um die Wirbelfähigkeit des Katalysators zu gewährleisten.

Bevorzugt ist das nach dem erfindungsgemäßen Verfahren hergestellte aromatische Amin Anilin und die entsprechende Nitroverbindung Nitrobenzol.

Als Katalysatoren können die bekannten, partikelförmigen, geträgerten oder nichtgeträgerten Katalysatoren für die Hydrierung von aromatischen Aminen eingesetzt werden, insbesondere Katalysatoren enthaltend Schwermetalle der ersten und/oder der fünften bis achten Gruppe des Periodensystems, bevorzugt ein oder mehrere der Elemente Kupfer, Palladium, Molybdän, Wolfram, Nickel und Kobalt.

Die Zellen sind in ihrer Geometrie nicht eingeschränkt; es kann sich beispielsweise um Zellen mit runden Wänden, insbesondere Hohlkugeln, oder auch um Zellen mit eckigen Wänden handeln. Bei einer eckigen Ausbildung der Wände ist bevorzugt, dass die Zelle nicht mehr als 50 Ecken aufweisen, vorzugsweise nicht mehr als 30 und insbesondere nicht mehr als 10 Ecken.

Die Zellwände in den Zellen der Einbauten sind gasdurchlässig ausgebildet, um durch ein Durchströmen der Gasphase durch die Zellen das Wirbeln des heterogenen partikelförmigen Katalysators zu gewährleisten. Hierzu können die Zellwände aus einem Siebgewebe gebildet sein oder auch aus flächenförmigen Materialien, die beispielsweise runde oder auch anders geformte Löcher aufweisen.

Hierbei beträgt die mittlere Maschenweite der eingesetzten Siebgewebe oder die bevorzugte Weite der Löcher in den Zellwänden insbesondere zwischen 50 und 1 mm, weiter bevorzugt zwischen 10 und 1 mm und besonders bevorzugt zwischen 5 und 1 mm.

Beispiele für Kreuzkanalpackungen sind die Packungen der Typen Mellpack^{®}, CY oder BX der Fa. Sulzer AG, CH-8404 Winterthur oder die Typen A3, BSH, B1 oder M der Fa. Monz GmbH, D-40723 Hilden.

In den Kreuzkanalpackungen bilden sich in vertikaler Richtung zwischen zwei jeweils aufeinander folgenden Metallblechen, Streckmetall- oder Gewebelagen, durch die geknickte Strukturierung derselben, Hohlräume, das heißt Zellen aus, die durch Engstellen zwischen den Knickkanten begrenzt werden.

Der mittlere hydraulische Durchmesser der Zellen, bestimmt mittels radioaktiver Tracertechnik, und die beispielsweise vorstehend in Verbindung mit der Bestimmung der Austauschzahl angegebenen Literaturstelle beschrieben ist, liegt bevorzugt im Bereich zwischen 500 und 1 mm, weiter bevorzugt zwischen 100 und 5 mm und besonders bevorzugt zwischen 50 und 5 mm.

Hierbei wird als hydraulischer Durchmesser in bekannter Weise die vierfache horizontale Querschnittsfläche der Zelle, dividiert durch den von oben betrachteten Umfang der Zelle, definiert.

Die mittlere Höhe der Zellen, gemessen in vertikaler Richtung im Wirbelbettreaktor mittels radioaktiver Tracertechnik, beträgt vorzugsweise zwischen 100 und 1 mm, weiter bevorzugt zwischen 100 und 3 mm und besonders bevorzugt zwischen 40 und 5 mm.

Die obigen Kreuzkanalpackungen belegen nur einen geringen Teil der Querschnittsfläche des Wirbelschichtreaktors, insbesondere einen Anteil von etwa 1 bis 10 % derselben.

Bevorzugt liegen die Neigungswinkel der Knickflächen zur Vertikalen im Bereich von 10 bis 80°, insbesondere zwischen 20 und 70°, besonders bevorzugt zwischen 30 und 60°.

Die Knickflächen in den Metallblechen, Streckmetall- oder Gewebelagen weisen bevorzugt eine Knickhöhe im Bereich zwischen 100 und 3 mm, besonders bevorzugt zwischen 40 und 5 mm, und einen Abstand der Engstellen zwischen den Knickkanten im Bereich zwischen 50 und 2 mm, besonders bevorzugt zwischen 20 und 3 mm, auf.

In die Zellen bildenden Einbauten können für eine gezielte Steuerung der Reaktionstemperatur Wärmeübertrager zwecks Zuführung von Wärme bei endothermen Reaktionen oder Abfuhr von Wärme bei exothermen Reaktionen eingebaut werden. Die Wärmeübertrager können beispielsweise plattenförmig oder rohrförmig ausgebildet sein und im Wirbelschichtreaktor vertikal, horizontal oder geneigt angeordnet sein.

Die Wärmeübertragungsflächen können auf die konkrete Reaktion abgestimmt werden; dadurch kann jede Reaktion wärmetechnisch mit dem erfindungsgemäßen Reaktorkonzept realisiert werden.

Die Zellen bildenden Einbauten werden bevorzugt aus Werkstoffen mit sehr guter Wärmeleitfähigkeit hergestellt, so dass der Wärmetransport über die Zellwände nicht behindert wird. Somit entsprechen die Wärmeübergangseigenschaften des erfindungsgemäßen Reaktors jenen eines herkömmlichen Wirbelschichtreaktors.

Die Werkstoffe für die Zellen bildenden Einbauten sollen darüber hinaus eine ausreichende Stabilität unter Reaktionsbedingungen aufweisen; insbesondere ist neben der Beständigkeit gegenüber chemischen und thermischen Belastungen auch die Beständigkeit des Werkstoffs gegenüber mechanischem Angriff durch den wirbelnden Katalysator zu beachten.

Aufgrund der einfachen Bearbeitbarkeit derselben sind Metall-, Keramik-, Polymer- oder Glaswerkstoffe besonders geeignet.

Die Einbauten sind bevorzugt dergestalt ausgebildet, dass sie 10 bis 90 Vol.-% der Wirbelschicht in Zellen unterteilen.

Hierbei ist bevorzugt der untere Bereich der Wirbelschicht in Durchströmungsrichtung des gasförmigen Reaktionsgemisches von Einbauten frei.

Besonders bevorzugt sind die Einbauten, die die Wirbelschicht in Zellen aufteilen, oberhalb der Wärmeübertrager angeordnet. Dadurch kann insbesondere der Restumsatz erhöht werden.

Durch die begrenzte Querschnittsbelegung durch die Zellen bildenden Einbauten weist der erfindungsgemäße Reaktor keine Nachteile hinsichtlich Entmischung und Austragsneigung des wirbelnden partikelförmigen Katalysators auf.

Die Erfindung wird im Folgenden anhand einer Zeichnung näher erläutert.

Es zeigen im Einzelnen:
- Figur 1: die schematische Darstellung einer bevorzugten Ausführungsform eines erfindungsgemäß eingesetzten Wirbelschichtreaktors, und
- Figur 2: die schematische Darstellung einer bevorzugten Ausführungsform von erfindungsgemäß eingesetzten Einbauten.

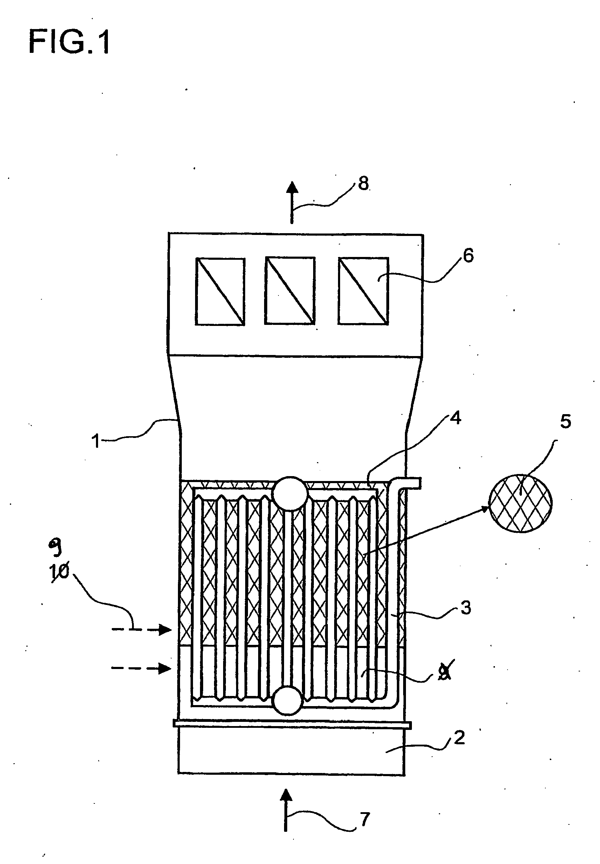

Der in Figur 1 dargestellte Wirbelschichtreaktor 1 umfasst eine feststofffreie Gasverteilerzone 2, Einbauten 4, die Zellen 5 ausbilden, mit einem Wärmeübertrager 3 im Bereich der Einbauten 4.

Oberhalb der Reaktionszone erweitert sich der Reaktor und weist mindestens einen Feststoffabscheider 6 auf. Der Pfeil 7 gibt die Zuführung der gasförmigen Einsatzstoffe und der Pfeil 8 die Abführung des gasförmigen Produktstromes an. Zusätzliche flüssigphasige Edukte können seitlich, über die gestrichelten Pfeile 9, zugegeben werden.

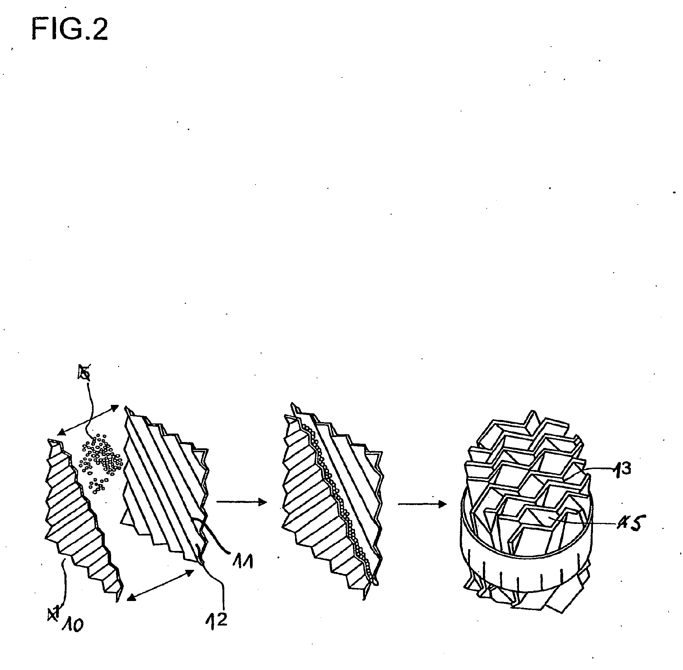

Figur 2 stellt eine bevorzugte Ausführungsform von erfindungsgemäßen Einbauten 4 in Form einer Kreuzkanalpackung dar, mit geknickten Metallblechen 10, die parallel zueinander in Längsrichtung angeordnet sind, mit Knickkanten 11, die das Metallblech 10 in Knickflächen 12 unterteilen und wobei zwei aufeinander folgende Metallbleche so angeordnet sind, dass sie den gleichen Neigungswinkel, jedoch mit umgekehrten Vorzeichen aufweisen und dabei Zellen 5 ausbilden, die in vertikaler Richtung durch Engstellen 13 begrenzt werden.

## Patentansprüche

1. Verfahren zur Herstellung von aromatischen Aminen durch katalytische Hydrierung der entsprechenden Nitroverbindung in einem Wirbelschichtreaktor (1), wobei ein gasförmiges Reaktionsgemisch (7), enthaltend die Nitroverbindung und Wasserstoff, einen heterogenen partikelförmigen, eine Wirbelschicht ausbildenden Katalysator von unten nach oben durchströmt, und wobei in der Wirbelschicht Einbauten (4) vorgesehen sind, die die Wirbelschicht in eine Mehrzahl von horizontal sowie eine Mehrzahl von vertikal im Wirbelschichtreaktor (1) angeordneten Zellen (5) aufteilen, mit Zellwänden, die gasdurchlässig sind und die Öffnungen aufweisen, die eine Austauschzahl des heterogenen, partikelförmigen Katalysators in vertikaler Richtung im Bereich von 1 bis 100 Litern/Stunde pro Liter Reaktorvolumen gewährleisten, **dadurch gekennzeichnet, dass** die Einbauten als Kreuzkanalpackung ausgebildet sind, mit in vertikaler Richtung im Wirbelschichtreaktor (1) parallel zueinander angeordneten geknickten gasdurchlässigen Metallblechen (10), Streckmetall- oder Gewebelagen, mit Knickkanten (11), die Knickflächen (12) mit einem von Null verschiedenen Neigungswinkel zur Vertikalen ausbilden, und wobei die Knickflächen (12) aufeinander folgender Metallbleche (10), Streckmetall- oder Gewebelagen den gleichen Neigungswinkel, jedoch mit umgekehrten Vorzeichen aufweisen und **dadurch** Zellen (5) ausbilden, die in vertikaler Richtung durch Engstellen (13) zwischen den Knickkanten (11) begrenzt werden.

2. Verfahren nach Anspruch 1, **dadurch gekennzeichnet, dass** das aromatische Amin Anilin und die entsprechende Nitroverbindung Nitrobenzol ist.

3. Verfahren nach Anspruch 1 oder 2, **dadurch gekennzeichnet, dass** als Katalysatoren geträgerte oder nicht-geträgerte Katalysatoren, enthaltend Schwermetalle der 1. und/oder der 5. bis 8. Gruppe des Periodensystems, insbesondere eines oder mehrere der Elemente Kupfer, Palladium, Molybdän, Wolfram, Nickel, Kobalt, eingesetzt werden.

4. Verfahren nach Anspruch 1, **dadurch gekennzeichnet, dass** die Neigungswinkel der Knickflächen (12) zur Vertikalen im Bereich von 10 bis 80°, bevorzugt zwischen 20 und 70°, besonders bevorzugt zwischen 30 und 60°, liegen.

5. Verfahren nach einem der Ansprüche 1 bis 3, **dadurch gekennzeichnet, dass** die Zellen (5) der Einbauten (4) einen hydraulischen Durchmesser, gemessen mittels radioaktiver Tracertechnik, zwischen 100 und 5 mm, bevorzugt zwischen 50 und 5 mm, aufweisen.

6. Verfahren nach einem der Ansprüche 1 bis 5, **dadurch gekennzeichnet, dass** in die Einbauten (4) Wärmeübertrager (3) eingebracht sind.

7. Verfahren nach Anspruch 6, **dadurch gekennzeichnet, dass** die Wärmeübertrager (3) platten- oder rohrförmig ausgebildet sind.

8. Verfahren nach einem der Ansprüche 1 bis 5, **dadurch gekennzeichnet, dass** die Einbauten (4) aus Metall-, Keramik-, Polymer- oder Glaswerkstoffen gebildet sind.

9. Verfahren nach einem der Ansprüche 1 bis 7, **dadurch gekennzeichnet, dass** die Einbauten (4) 10 bis 90 Volumenprozent der Wirbelschicht in Zellen (5) unterteilen.

10. Verfahren nach Anspruch 8, **dadurch gekennzeichnet, dass** der untere Bereich der Wirbelschicht in Durchströmungsrichtung des gasförmigen Reaktionsgemisches von Einbauten (4) frei ist.

11. Verfahren nach Anspruch 6 oder 7, **dadurch gekennzeichnet, dass** die Einbauten (4), die die Wirbelschicht in Zellen (5) aufteilen, oberhalb der Wärmeübertrager (3) angeordnet sind.

## Claims

1. A process for preparing aromatic amines by catalytic hydrogenation of the corresponding nitro compound in a fluidized-bed reactor (1), in which a gaseous reaction mixture (7) comprising the nitro compound and hydrogen flows from the bottom upward through a heterogeneous particulate catalyst forming a fluidized bed and in which the fluidized bed is provided with internals (4) which divide the fluidized bed into a plurality of cells (5) arranged horizontally in the fluidized-bed reactor (1) and a plurality of cells (5) arranged vertically in the fluidized-bed reactor (1), with the cells having cell walls which are permeable to gas and have openings which ensure an exchange number of the heterogeneous, particulate catalyst in the vertical direction in the range from 1 to 100 liters/hour per liter of reactor volume, wherein the internals are configured as cross-channel packing having creased gas-permeable metal sheets (10), expanded metal sheets or woven meshes which are arranged in parallel to one another in the vertical direction in the fluidized-bed reactor (1) and have creases (11) which form flat areas (12) between the creases having an angle of inclination to the vertical which is different from zero, with the flat areas (12) between the creases of successive metal sheets (10), expanded metal sheets or woven meshes having the same angle of inclination but with the opposite sign so as to form cells (5) which are delimited in the vertical direction by constrictions (13) between the creases (11).

2. The process according to claim 1, wherein the aromatic amine is aniline and the corresponding nitro compound is nitrobenzene.

3. The process according to claim 1 or 2, wherein supported or unsupported catalysts comprising heavy metals of the 1st and/or 5th to 8th group of the Periodic Table, in particular one or more of the elements copper, palladium, molybdenum, tungsten, nickel, cobalt, are used as catalysts.

4. The process according to claim 1, wherein the angle of inclination to the vertical of the flat areas (12) between the creases is in the range from 10 to 80°, preferably from 20 to 70°, particularly preferably from 30 to 60°.

5. The process according to any of claims 1 to 3, wherein the cells (5) of the internals (4) have a hydraulic diameter measured by means of the radioactive tracer technique of from 100 to 5 mm, preferably from 50 to 5 mm.

6. The process according to any of claims 1 to 5, wherein heat exchangers (3) are installed in the internals (4).

7. The process according to claim 6, wherein the heat exchangers (3) are configured in the form of plates or tubes.

8. The process according to any of claims 1 to 5, wherein the internals (4) are made of metal, ceramic, polymer or glass materials.

9. The process according to any of claims 1 to 7, wherein the internals (4) divide from 10 to 90% by volume of the fluidized bed into cells (5).

10. The process according to claim 8, wherein the lower region of the fluidized bed in the flow direction of the gaseous reaction mixture is free of internals (4).

11. The process according to claim 6 or 7, wherein the internals (4) which divide the fluidized bed into cells (5) are located above the heat exchangers (3).

## Revendications

1. Procédé de fabrication d'amines aromatiques par hydrogénation catalytique du composé nitro correspondant dans un réacteur à lit fluidisé (1), un mélange réactionnel gazeux (7), qui contient le composé nitro et de l'hydrogène, traversant du bas vers le haut un catalyseur hétérogène particulaire qui forme un lit fluidisé, et des composants internes (4) étant prévus dans le lit fluidisé, qui divisent le lit fluidisé en une pluralité de cellules (5) disposées horizontalement et verticalement dans le réacteur à lit fluidisé (1), les parois desdites cellules étant perméables aux gaz et comportant des ouvertures qui assurent un taux d'échange du catalyseur hétérogène particulaire dans la direction verticale dans la plage allant de 1 à 100 litres/heure par litre du volume du réacteur, **caractérisé en ce que** les composants internes forment un garnissage en canaux croisés, avec, dans la direction verticale dans le réacteur à lit fluidisé (1), des tôles métalliques (10), des couches de métal déployé ou de tissu, perméables aux gaz, pliées et placées parallèlement les unes aux autres, avec des arêtes de pliage (11) qui forment des surfaces de pliage (12) ayant un angle d'inclinaison par rapport à la verticale différent de zéro, les surfaces de pliage (12) de tôles métalliques (10), couches de métal déployé ou de tissu, successives présentant le même angle d'inclinaison, avec toutefois des signes inverses, et formant ainsi des cellules (5) qui sont limitées dans la direction verticale par des points étroits (13) entre les arêtes de pliage (11).

2. Procédé selon la revendication 1, **caractérisé en ce que** l'amine aromatique est l'aniline et le composé nitro correspondant est le nitrobenzène.

3. Procédé selon la revendication 1 ou 2, **caractérisé en ce que** des catalyseurs supportés ou non supportés, contenant des métaux lourds du groupe 1 et/ou 5 à 8 du tableau périodique, notamment un ou plusieurs des éléments cuivre, palladium, molybdène, tungstène, nickel, cobalt, sont utilisés en tant que catalyseurs.

4. Procédé selon la revendication 1, **caractérisé en ce que** l'angle d'inclinaison des surfaces de pliage (12) par rapport à la verticale se situe dans la plage allant de 10 à 80°, de préférence est compris entre 20 et 70°, de manière particulièrement préférée entre 30 et 60°.

5. Procédé selon l'une quelconque des revendications 1 à 3, **caractérisé en ce que** les cellules (5) des composants internes (4) présentent un diamètre hydraulique, mesuré par une technique de traçage radioactif, compris entre 100 et 5 mm, de préférence entre 50 et 5 mm.

6. Procédé selon l'une quelconque des revendications 1 à 5, **caractérisé en ce que** des échangeurs de chaleur (3) sont placés dans les composants internes (4).

7. Procédé selon la revendication 6, **caractérisé en ce que** les échangeurs de chaleur (3) sont de forme plate ou tubulaire.

8. Procédé selon l'une quelconque des revendications 1 à 5, **caractérisé en ce que** les composants internes (4) sont formés à partir de matériaux métalliques, céramiques, polymères ou de verre.

9. Procédé selon l'une quelconque des revendications 1 à 7, **caractérisé en ce que** les composants internes (4) divisent 10 à 90 pourcent en volume du lit fluidisé en cellules (5).

10. Procédé selon la revendication 8, **caractérisé en ce que** la zone inférieure du lit fluidisé dans la direction de l'écoulement du mélange réactionnel gazeux est exempte de composants internes (4).

11. Procédé selon la revendication 6 ou 7, **caractérisé en ce que** les composants internes (4) qui divisent le lit fluidisé en cellules (5) sont placés au-dessus des échangeurs de chaleur (3).
